# EUROPÄISCHE PATENTSCHRIFT

(11) **EP 2 084 545 B1**
(45) Veröffentlichungstag und Bekanntmachung des Hinweises auf die Patenterteilung: **12.02.2014**
(21) Anmeldenummer: 07817601.3
(22) Anmeldetag: 01.10.2007
(51) Int. Cl.: G01N 33/74

(54) **DIAGNOSE VON INFEKTIONEN ODER ENTZÜNDUNGSERKRANKUNGEN DER ATEMWEGE UND LUNGE ASSOZIIERT MIT HERZINSUFFIZIENZ**
DIAGNOSIS OF INFECTIONS OR INFLAMMATORY DISEASES OF THE AIRWAYS AND LUNGS ASSOCIATED WITH HEART FAILURE
DIAGNOSTIC D'INFECTIONS OU DE MALADIES INFLAMMATOIRES DES VOIES RESPIRATOIRES ET DES POUMONS ASSOCIÉS À L'INSUFFISANCE CARDIAQUE

(30) Priorität: 01.10.2006 DE 102006046996
(43) Veröffentlichungstag der Anmeldung: 05.08.2009
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE)
(74) Vertreter: Kilger, Ute
(86) Internationale Anmeldenummer: PCT/DE2007/001759
(87) Internationale Veröffentlichungsnummer: WO 2008/040328

(56) Entgegenhaltungen:
- SANDEK A ET AL: "Procalcitonin-guided antibiotic treatment in heart failure" LANCET THE, LANCET LIMITED. LONDON, GB, Bd. 363, Nr. 9420, 8. Mai 2004 (2004-05-08), Seite 1555, XP004769809 ISSN: 0140-6736
- CHRIST-CRAIN M ET AL: "Effect of procalcitonin-guided treatment on antibiotic use and outcome in lower respiratory tract infections: cluster-randomised, single-blinded intervention trial" LANCET THE, LANCET LIMITED. LONDON, GB, Bd. 363, Nr. 9409, 21. Februar 2004 (2004-02-21), Seiten 600-607, XP004747009 ISSN: 0140-6736
- REMSKAR ET AL: "Procalcitonin in patients with acute myocardial infarction" WIENER KLINISCHE WOCHENSCHRIFT, NEW YORK, NY, US, Bd. 114, Nr. 5-6, 2002, Seiten 205-210, XP009097045 ISSN: 0043-5325
- M. Christ-Crain: "Procalcitonin Guidance of Antibiotic Therapy in Community-acquired Pneumonia: A Randomized Trial", American Journal of Respiratory and Critical Care Medicine, vol. 174, no. 1, 1 January 2006 (2006-01-01), pages 84-93, XP55021728, ISSN: 1073-449X, DOI: 10.1164/rccm.200512-1922OC
- Anthony K Akobeng: "Understanding diagnostic tests 3: receiver operating characteristic curves", Acta Paediatrica, vol. 96, no. 5, 1 May 2007 (2007-05-01), pages 644-647, XP55036899, ISSN: 0803-5253, DOI: 10.1111/j.1651-2227.2006.00178.x
- MAISEL A S ET AL: "RAPID MEASUREMENT OF B-TYPE NATRIURETIC PEPTIDE IN THE EMERGENCY DIAGNOSIS OF HEART FAILURE", NEW ENGLAND JOURNAL OF MEDICINE, THE, MASSACHUSETTS MEDICAL SOCIETY, WALTHAM, MA, US, vol. 347, no. 3, 18 July 2002 (2002-07-18) , pages 161-167, XP009074659, ISSN: 0028-4793, DOI: 10.1056/NEJMOA020233

## Beschreibung

Die Erfindung betrifft ein Verfahren zur in vitro Diagnose von bakterieller Pneumonie mit assoziierter Herzinsuffizienz, wobei eine Bestimmung des Markers Procalcitonin oder eine Teilsequenz davon an einem zu untersuchenden Patienten durchgeführt wird, insbesondere zur Risikostratifizierung von Patienten.

In Europa kommen jährlich etwa eine Million Patienten mit dem Symptom der akuten Atemnot in die Notaufnahmen von Kliniken. Die Atemnot ist ein Leitsymptom vieler Erkrankungen und lässt sich in ca. 35-47% der Fälle auf eine Herzinsuffizienz (Januzzi JL Jr, Camargo CA, Anwaruddin S, Baggish AL, Chen AA, Krauser DG, Tung R, Cameron R, Nagurney JT, Chae CU, Lloyd-Jones DM, Brown DF, Foran-Melanson S, Sluss PM, Lee-Lewandrowski E, Lewandrowski KB, The N-terminal Pro-BNP investigation of dyspnea in the emergency department (PRIDE) study, Am J Cardiol. 95(8) (2005), pp. 948-954 und Maisel AS, Krishnaswamy P, Nowak RM, McCord J, Hollander JE, Duc P, Omland T, Storrow AB, Abraham WT, Wu AH, Clopton P, Steg PG, Westheim A, Knudsen CW, Perez A, Kazanegra R, Herrmann HC, McCullough PA; Breathing Not Properly Multinational Study Investigators, Rapid measurement of B-type natriuretic peptide in the emergency diagnosis of heart failure, N Engl J Med. 347(3) (2002), pp. 161-167) und in ca. 11% der Fälle auf eine Pneumonie (Januzzi JL et al, 2005 (supra)) zurückführen. Da Herzinsuffizienz ein entscheidender Risikofaktor für die Entstehung von Pneumonien ist (Huntemann I, Lorenz J, Ambulant erworbene Pneumonie (AEP), Übersichtsartikel auf der CAPNETZ-Webseite: www.capnetz.de), können beide Krankheiten miteinander assoziiert sein, und ein Teil der Patienten, der sich in der Notaufnahme vorstellt, leidet sowohl an einer Pneumonie als auch an einer Herzinsuffizienz (Christ-Crain M, Stolz D, Bingisser R, Muller C, Miedinger D, Huber PR, Zimmerli W, Harbarth S, Tamm M, Muller B, Procalcitonin Guidance of Antibiotic Therapy in Community-acquired Pneumonia: A Randomized Trial, Am J Respir Crit Care Med 174(1) (2006), pp. 84-93).

Um mit einer geeigneten Therapie zu beginnen, bedarf es einer frühen Diagnose und Differenzierung der zugrunde liegenden Erkrankung/Erkrankungen bereits in der Notaufnahme. Aufgrund unspezifischer Symptome (Luftnot, Husten) bei beiden Erkrankungen (Herzinsuffizienz und Pneumonie) ist sowohl die Differenzierung und Abgrenzung beider Erkrankungen als auch die Erkennung des gleichzeitigen Auftretens häufig erschwert (Huntemann I et al. (supra) und Maisel AS et al, 2002 (supra)). Um die Diagnostik zu erleichtern werden als Laboruntersuchungen die Bestimmung des B-Typ natriuretischen Peptids (BNP) bzw. seiner Prohormonfragmente wie NT-proBNP (Januzzi JL et al, 2005 (supra) und Maisel AS et al, 2002 (supra)) und des C-reaktiven Proteins durchgeführt. Ein niedriger BNP-Wert von <100 pg/ml kann zum Ausschluss der Diagnose Herzinsuffizienz führen (Maisel AS et al, 2002 (supra) und Ray P, Lefort Y, Usefulness of B-type natriuretic peptide in emergency medicine, Rev Med Interne 27 (2006), Epub ahead of print), ein sehr hoher Wert >500 pg/ml macht das Vorliegen einer Herzinsuffizienz wahrscheinlich (Ray P et al. 2006 (supra)). Schwieriger gestaltet es sich mit der Bewertung des CRP zur Bestätigung oder zum Ausschluss einer Pneumonie. Einerseits kann es unspezifisch bei einer Reihe nicht durch Erreger bedingter Erkrankungen (Autoimmunerkrankungen, Gewebsnekrosen, Herzinfarkt) erhöht sein (Wicher J, C-reaktives Protein (CRP) in Labor und Diagnose, Indikation und Bewertung von Laborbefunden für die medizinische Diagnostik, herausgegeben von Lothar Thomas 1998, 5. Auflage, TH-Books Verlagsgesellschaft 1998). Andererseits kann die Herzinsuffizienz selbst eine Erhöhung des CRP verursachen (Kardys I, Knetsch AM, Bleumink GS, Deckers JW, Hofman A, Stricker BH, Witteman JC, C-reactive protein and risk of heart failure. The Rotterdam Study, Am Heart J. 152(3) (2006): pp. 514-520). Aus diesen Gründen ist es schwierig auch mit Hilfe der üblichen Laboruntersuchungen bei Patienten mit einer Herzinsuffizienz das zusätzliche Vorliegen einer Pneumonie festzustellen. Dies ist aber von großer Bedeutung, da wie bereits oben erwähnt das Vorliegen einer Herzinsuffizienz eine Pneumonie begünstigt und die frühe Diagnose und die dadurch frühe therapeutische Behandlung einer Pneumonie mit Antibiotika die Prognose der Patienten erheblich verbessert (Welte T, Community-acquired and nosocomial pleumonia, Internist (Berl) 44 (2003), pp 44-58) und zu einer Reduktion der Behandlungskosten führt.

Im Stand der Technik ist die Procalcitonin (PCT)-Bestimmung beschrieben zwecks Untersuchung zur Abgrenzung einer bakteriellen Sepsis (Schwellenwert >0,5 ng/mL) von anderen Krankheitsursachen (EP0656121). PCT ist ebenfalls in Zusammenhang mit Pneumonien in der Literatur beschrieben, wobei sich die Untersuchungen hinsichtlich der Diagnose vor allem auf die Diskriminierung von unterschiedlichen Erregertypen bei bereits diagnostizierter Pneumonie beziehen (Prat C, Dominguez J, Andreo F, Blanco S, Pallares A, Cuchillo F, Ramil C, Ruiz-Manzano J, Ausina V, Procalcitonin and neopterin correlation with aetiology and severity of pneumonia, J Infect. 52(3) (2006): pp169-177 und Masia M, Gutierrez F, Shum C, Padilla S, Navarro JC, Flores E, Hernandez I, Masia M, Gutierrez F, Shum C, Padilla S, Navarro JC, Flores E, Hernandez I, Chest 128(4) (2005): pp 2223-2239, Boussekey N, Leroy O, Georges H, Devos P, d'Escrivan T, Guery B, Diagnostic and prognostic values of admission procalcitonin levels in community-acquired pneumonia in an intensive care unit. Infection 33(4) (2005): pp 257-63). In einer Arbeit von Zhou et al (Zhou CD et al Zhongguo Wei Zhong Bing Ji Jiu Yi Xue. 2006 Jun,18(6),370-2) wird PCT als diagnostischer Marker für die frühe Diagnose einer Ventilator-assoziierten Pneumonie auf einer Intensivstation vorgestellt. Ebenfalls als prognostischer Marker ist das PCT bei Pneumonien beschrieben (Prat et al. 2006 (supra) und Masia M et al. 2005 (supra), Boussekey N et al. 2005 (supra), Christ-Crain M, Morgenthaler NG, Stolz D, Muller C, Bingisser R, Harbarth S, Tamm M, Struck J, Bergmann A, Muller B, Pro-adrenomedullin to predict severity and outcome in community-acquired pneumonia, Crit Care 10(3) (2006): pp R96). Es liegen zudem Untersuchungen vor, bei denen gezeigt wurde, dass mit Hilfe von PCT bei Patienten mit Verdacht auf Infektionen der unteren Atemwege (einschließlich Pneumonien) bei einer Schwellenwertkonzentration von >0,1 ng/mL bzw. >0,25 ng/mL klinisch relevante Infektionen (darunter ebenfalls bakterielle Pneumonien), die einer Antibiotikatherapie bedürfen, detektiert werden (Christ-Crain M, Stolz D, Bingisser R, Muller C, Miedinger D, Huber PR, Zimmerli W, Harbarth S, Tamm M, Muller B, Procalcitonin Guidance of Antibiotic Therapy in Community-acquired Pneumonia: A Randomized Trial, Am J Respir Crit Care Med 174(1) (2006), pp. 84-93 und Christ-Crain M, Jaccard-Stolz D, Bingisser R, Gencay MM, Huber PR, Tamm M, Muller B, Effect of procalcitonin-guided treatment on antibiotic use and outcome in lower respiratory tract infections: cluster-randomised, single-blinded intervention trial, Lancet 21;363(9409) (2004): pp 600-607, Stolz D, Christ-Crain M, Gencay MM, Bingisser R, Huber PR, Muller B, Tamm M, Diagnostic value of signs, symptoms and laboratory values in lower respiratory tract infection, Swiss Med Wkly 8;136(27-28) (2006) : pp 434-440).

Nicht bekannt ist jedoch ein Verfahren zur Diagnose von Infektionen oder Entzündungserkrankungen der Atemwege und Lunge mit assoziierter Herzinsuffizienz.

Daher ist es Aufgabe der vorliegenden Erfindung ein Verfahren zur Diagnose von Infektionen oder Entzündungserkrankungen der Atemwege und Lunge mit assoziierter Herzinsuffizienz bereitzustellen.

Die Aufgabe wird durch ein Verfahren zur in vitro Diagnose bakterieller Pneumonie mit assoziierter Herzinsuffizienz gelöst, wobei eine Bestimmung des Markers Procalcitonin (PCT) oder eine Teilsequenz davon an einem zu untersuchenden Patienten durchgeführt wird (nachstehend erfindungsgemäßes Verfahren).

In einer bevorzugten Ausführungsform des erfindungsgemäßen Verfahrens ist der signifikante Bereich 0,01 ng/mL bis 1 ng/mL bestimmtes Procalcitonin (PCT) mit einem Schwellenwert zwischen 0,03 ng/mL und 0,25 ng/mL PCT (ROC-Kurven, vgl. Beispiele und Figuren).

Der Begriff "bakterielle Pneumonie" umfasst insbesondere die Komorbidität dieser Indikationen, d.h. es wird zusätzlich zu einer vorhandenen Grunderkrankung (Indexerkrankung) nämlich der Herzinsuffizienz ein vorliegendes, diagnostisch abgrenzbares Krankheitsbild nämlich der bakteriellen Pneumonie festgestellt bzw. es liegt ein assoziiertes Krankheitsbild vor, die insbesondere aufgrund ähnlicher Symptomatik (Atemnot, Brustschmerzen) zu einer Fehldiagnose oder Fehlinterpretation führen kann, da das eine Krankheitsbild das andere Krankheitsbild überdeckt.

Besonders vorteilhaft kann insbesondere in Fällen der Notfall- und /oder Intensivmedizin mittels des erfindungsgemäßen Verfahren eine sichere Diagnose erfolgen. Das erfindungsgemäße Verfahren ermöglicht klinische Entscheidungen, die zu einem schnellen Therapieerfolg führen. Solche klinische Entscheidungen umfassen ebenfalls weiterführende Behandlung mittels Arzneimitteln zur Behandlung oder Therapie von Herzinsuffizienz und zur Behandlung oder Therapie von bakterieller Pneumonie.

In einer weiteren bevorzugten Ausführungsform betrifft das erfindungsgemäße Verfahren daher die Therapiesteuerung zur Antibiotikabehandlung einer bakteriellen Pneumonie.

Daher betrifft die Erfindung ebenfalls ein Verfahren zur Risikostratifizierung von Patienten, insbesondere zur Stratifizierung von Patienten für klinische Entscheidungen, vorzugsweise in der zeitlich kritischen Intensivmedizin oder Notfallmedizin.

In einer weiteren bevorzugten Ausführungsform des erfindungsgemäßen Verfahrens erfolgt die Diagnose zur Prophylaxe, zur differentialdiagnostischen Früherkennung und Erkennung, zur Beurteilung des Schweregrades und zur therapiebegleitenden Verlaufsbeurteilung einer bakteriellen Pneumonie mit assoziierter Herzinsuffizienz.

In einer weiteren Ausführungsform des erfindungsgemäßen Verfahrens wird dem zu untersuchenden Patienten Blut entnommen, wahlweise Vollblut oder Serum, und die Diagnose erfolgt *in vitro*/*ex vivo,* d.h. außerhalb des menschlichen oder tierischen Körpers. Aufgrund der Bestimmung des Markers Procalcitonin und seiner vorhandenen Menge in mindestens einer Patientenprobe kann die Diagnose erfolgen.

Im Rahmen dieser Erfindung wird unter "Herzinsuffizienz" ein akutes oder chronisches Unvermögen des Herzens, die Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, deren ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Christian Mewis, Reimer Riessen und Ioakim Spyridopoulos, 2. unveränderte Auflage, Thieme 2006).

Im Rahmen dieser Erfindung wird unter Pneumonie (Lungenentzündung) eine akute oder chronische Entzündung des Lungengewebes verstanden und dessen Infektion verursacht durch Bakterien. Für den Kliniker ist die Pneumonie eine Konstellation verschiedener Symptome (Fieber oder Hypothermie, Schüttelfrost, Husten, pleuritischer Thoraxschmerz, gesteigerte Sputumproduktion, erhöhte Atemfrequenz, Klopfschalldämpfung, Bronchialatmen, ohrnahe Rasselgeräusche, Pleurareiben) in Kombination mit mindestens einem auf dem Thorax-Röntgenbild erkennbaren Infiltrat (Harrisons Innere Medizin, herausgegeben von Manfred Dietel, Norbert Suttorp und Martin Zeitz, ABW Wissenschaftsverlag 2005.

Alle genannten Indikationen werden zudem z.B. im Pschyrembel, De Gruyter, Berlin 2004 beschrieben.

Im Rahmen dieser Erfindung wird unter "Procalcitonin" ein humanes Protein oder Polypeptid verstanden mit einer Aminosäuresequenz von 1-116 AS oder 2-116 AS (PCT 2-116) oder 3-116 AS (PCT 3-116), wie in der EP0656121, EP1121600 der Anmelderin sowie DE10027954A1 beschrieben. Ferner kann das erfindungsgemäße Procalcitonin postranslationale Modifikationen aufweisen, wie Glykolisierung, Lip(o)idisierung oder Derivatisierungen. Ferner umfasst sind ebenfalls Teilsequenzen oder Fragmente von Procalcitonin.

In einer weiteren Ausführungsform kann die Bestimmung von Procalcitonin zusätzlich mit weiteren Markern erfolgen und zwar vorzugsweise solche, die bereits auf eine Herzinsuffizienz oder bakterielle Pneumonie hinweisen.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, und Adhäsionsmolekülen, wie VCAM oder ICAM, GDF-15 oder ST2 ausgewählt sein sowie der cardiovaskuläre Marker aus mindestens einem Marker aus der Gruppe Kreatinkinase, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin oder pro-Adrenomedullin (proADM) oder jeweils eine Teilsequenz davon.

Der ischämische Marker kann aus mindestens einem Marker aus der Gruppe Troponin I und T, CK-MB ausgewählt sein. Darüber hinaus kann der neurohormonaler Marker mindestens ein natriuretisches Protein sein, insbesondere ANP (bzw. ANF), proANP, NT-proANP, BNP, proBNP, NT-proBNP oder jeweils eine Teilsequenz davon.

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung.

Ferner betrifft die Erfindung die Verwendung von Procalcitonin oder einer Teilsequenz davon und ggfs. weitere Marker, wie oben ausgeführt, zur Diagnose von Infektionen oder Entzündungserkrankungen der Atemwege und Lunge mit assoziierter Herzinsuffizienz.

Nachfolgende Beispiele und Figuren dienen zur näheren Erläuterung der Erfindung, jedoch ohne die Erfindung auf diese Beispiele und Figuren zu beschränken.

### Beispiele und Figuren:

### Beispiel 1

Patienten, die sich mit dem Leitsymptom der Atemnot in der Notaufnahme eines Krankenhauses vorgestellt haben, wurde während der Eingangsuntersuchung eine Blutprobe entnommen. Durch Zentrifugation gewonnenes EDTA-Plasma wurde aliquotiert und bis zur Vermessung von PCT bei -80°C gelagert. PCT wurde mit dem Assay B·R·A·H·M·S PCT sensitive LIA (BRAHMS AG, Hennigsdorf, Deutschland, (Morgenthaler NG, Struck J, Fischer-Schulz C, Bergmann A, Sensitive Immunoluminometric Assay for the Detection of Procalcitonin, Clin Chem 48 (2002), pp. 788-790) vermessen. Der Assay verfügt über eine analytische Assaysensitivität von 0,01 ng/mL.

Die Diagnose der Herzinsuffizienz bei den untersuchten Patienten basierte auf dem Framingham Score (McKee PA, Castelli WP, McNamara P and Kannel WB, The natural history of congestive heart failure: the Framingham study, N Engl J Med 285 (1971), pp. 1441-1446) für Herzinsuffizienz plus einer echiographischen Evidenz für eine systolische oder diastolische Dysfunktuktion.

Pneumonie wurde diagnostiziert, wenn ein vorher nicht bekanntes Infiltrat im Röntgenbild beobachtet wurde und mindestens 2 respiratorische Symptome (Husten, Dyspnoe oder purtider Auswurf) aufgetreten waren.

Des Weiteren wurde gesunden Individuen ohne bekannte Erkrankung Blut abgenommen und durch Zentrifugation EDTA-Plasma gewonnen. Die Proben wurden bis zur PCT-Messung (B·R·A·H·M·S PCT sensitive LIA (Brahms AG, Hennigsdorf, Deutschland)) bei -20°C gelagert.

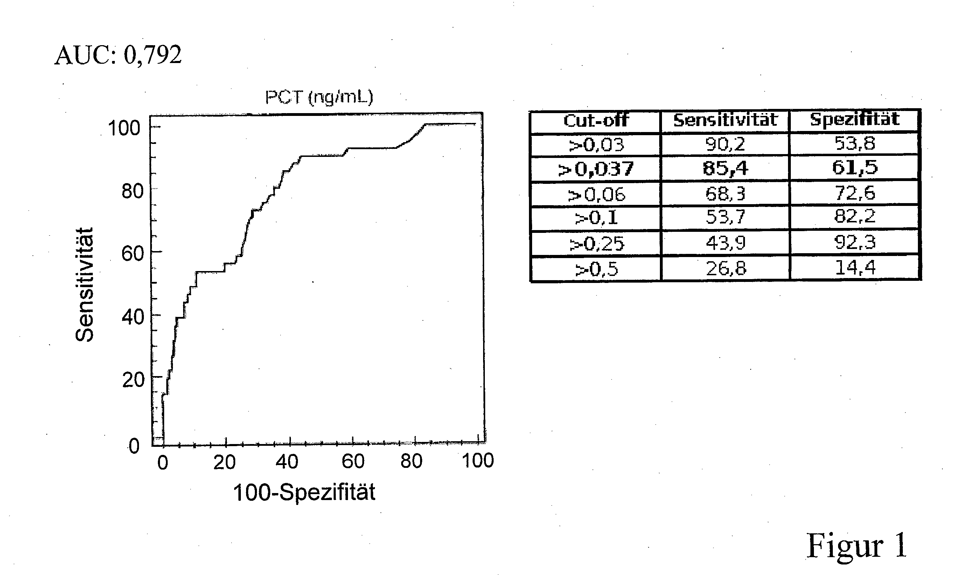

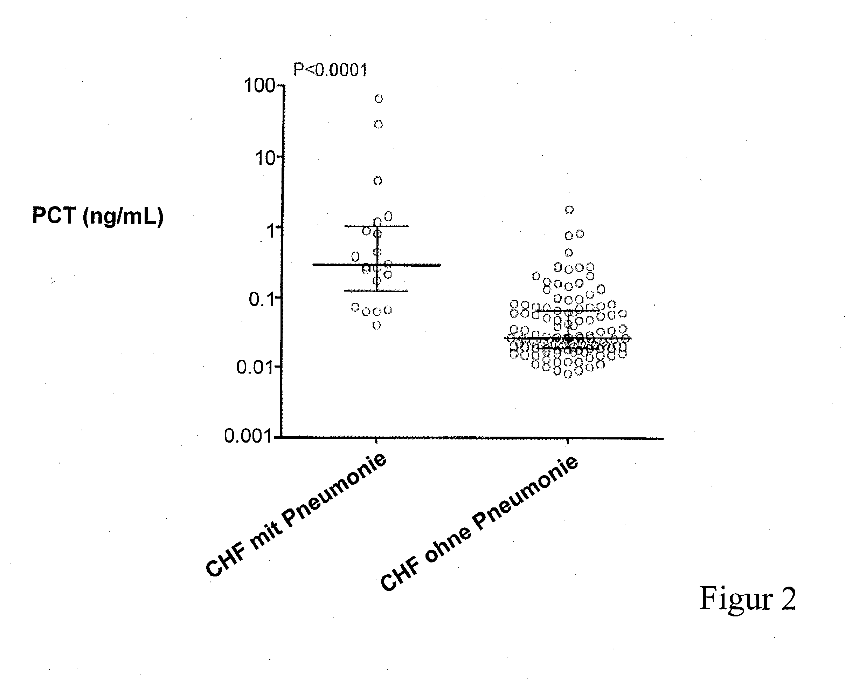

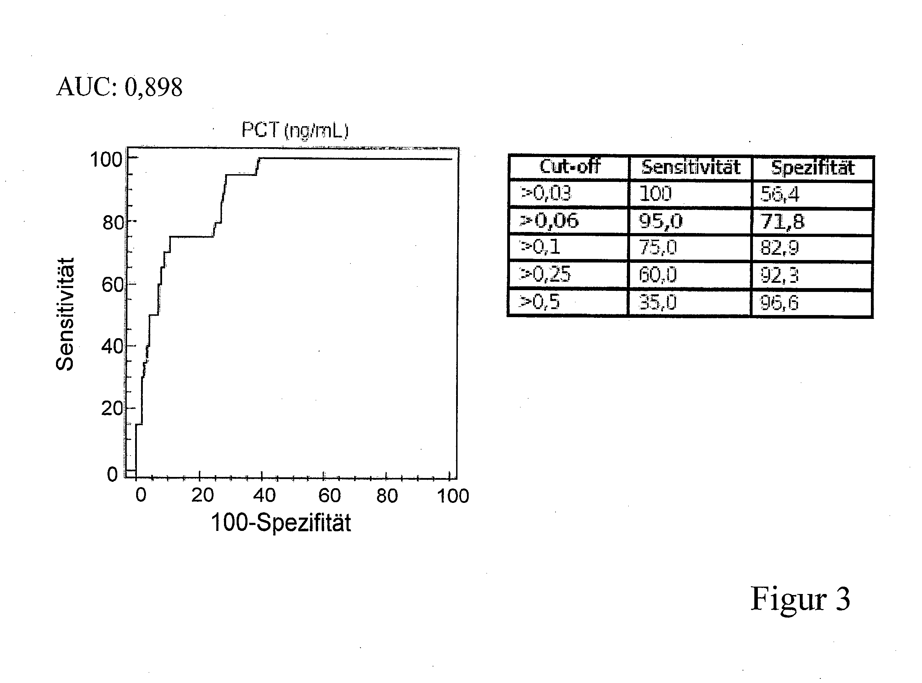

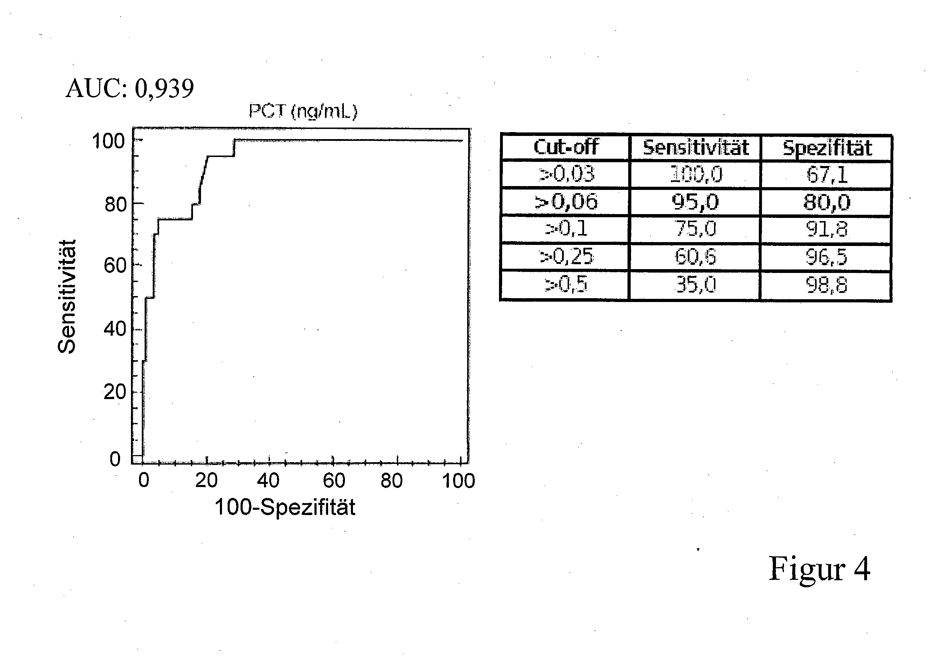

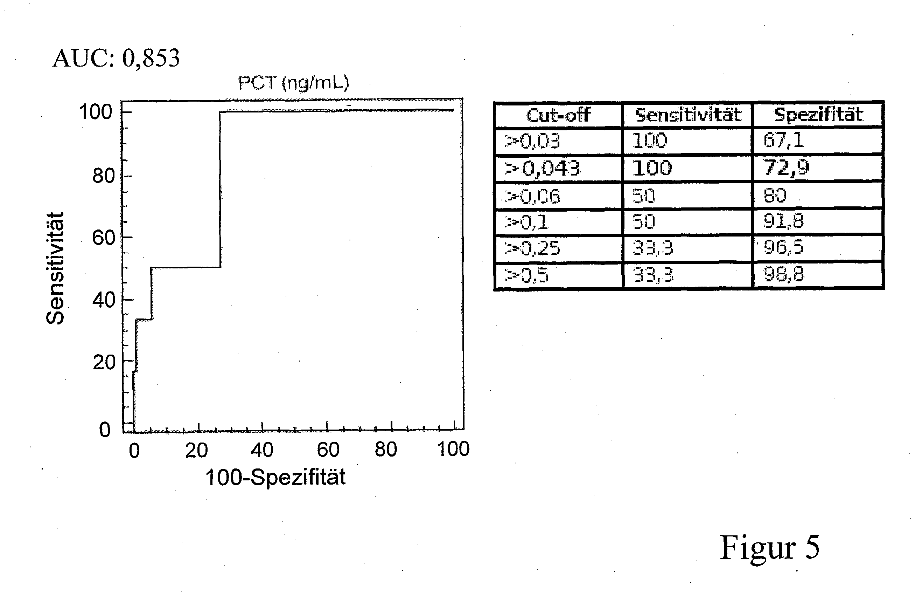

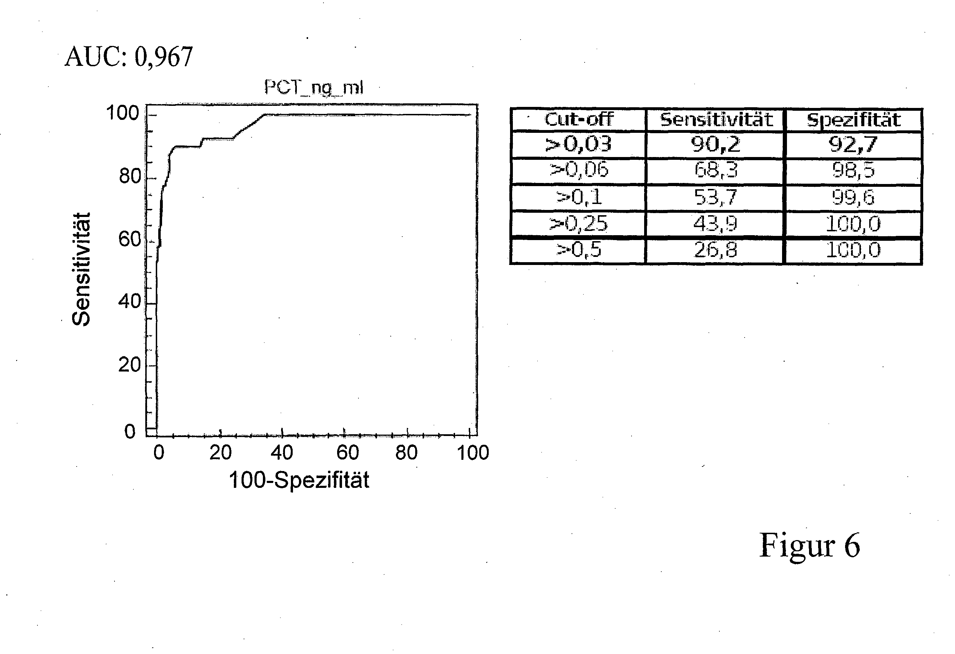

Figuren und Tabellenbeschriftung:
Figur 1:
   Links: ROC-Kurve für die Diagnose Pneumonie durch PCT bei Patienten mit Atemnot auf der Notaufnahme. Patienten ohne Pneumonie: n=208; Patienten mit der alleinigen Diagnose Pneumonie: n=21; Patienten mit Diagnose Pneumonie und Herzinsuffizienz: n=20. Die Area under the curve beträgt 0,792 (95% CI 0,736-0,840; p=0,0001).
   Rechts: Sensitivität und Spezifität für die Diagnose Pneumonie durch PCT bei verschiedenen Schwellenwert-Konzentrationen (cut-off [ng/mL]). Größte diagnostische Genauigkeit bei hervorgehobenem Schwellenwert.
Figur 2:
   Verteilung der PCT-Meßwerte bei Atemnotpatienten mit Herzinsuffizienz (CHF). Vergleich von Patienten mit und ohne Komorbidität Pneumonie. Gezeigt sind Einzelwerte mit Median und Interquartilbereich. Mann-Whitney U Test: signifikanter (P<0,0001) Unterschied zwischen beiden Gruppen.
Figur 3:
   Links: ROC-Kurve für die Diagnose Pneumonie durch PCT bei Patienten mit Herzinsuffizienz auf der Notaufnahme.
   Herzinsuffizienzpatienten ohne Pneumonie: n=117; Patienten mit Diagnose Pneumonie und Herzinsuffizienz: n=20. Die Area under the curve beträgt 0,898 (95% CI 0,835-0,943; p=0,0001).
   Rechts: Sensitivität und Spezifität für die Diagnose Pneumonie durch PCT bei verschiedenen Schwellenwert-Konzentrationen (cut-off [ng/mL]). Größte diagnostische Genauigkeit bei hervorgehobenem Schwellenwert.
Figur 4:
   Links: ROC-Kurve für die Diagnose Pneumonie durch PCT bei Patienten mit Herzinsuffizienz auf der Notaufnahme. Im Vergleich zu Figur 3 bezieht sich die Untersuchung nur auf Herzinsuffizienz-Patienten ohne Komorbidität und solche mit Pneumonie; Herzinsuffizienz-Patienten ohne Komorbidität: n=85; Patienten mit Diagnose Pneumonie und Herzinsuffizienz: n=20. Die Area under the curve beträgt 0,939 (95% CI 0,874-0,976; p=0,0001).
   Rechts: Sensitivität und Spezifität für die Diagnose Pneumonie durch PCT bei verschiedenen Schwellenwert-Konzentrationen (cut-off [ng/mL]). Größte diagnostische Genauigkeit bei hervorgehobenem Schwellenwert.
Figur 5:
   Links: ROC-Kurve für die Diagnose putride Bronchitis durch PCT bei Patienten mit Herzinsuffizienz auf der Notaufnahme. Herzinsuffizienzpatienten ohne Komorbidität: n=85; Patienten mit Diagnose putride Bronchitis: n=6. Die Area under the curve beträgt 0,853 (95% CI 0,763-0,918; p=0,0004).
   Rechts: Sensitivität und Spezifität für die Diagnose putride Bronchitis durch PCT bei verschiedenen Schwellenwert-Konzentrationen (cut-off [ng/mL]). Größte diagnostische Genauigkeit bei hervorgehobenem Schwellenwert.
Figur 6:
   Links: ROC-Kurve für die Diagnose Pneumonie durch PCT. Die ROC-Analyse bezieht sich auf den Vergleich von gesunden Kontrollindividuen und Patienten, die sich mit dem Symptom der Atemnot auf der Notaufnahme vorgestellt haben und für die eine Pneumonie diagnostiziert wurde. Patienten mit Diagnose Pneumonie: n=41, gesunde Kontrollindividuen: n=206. Die Area under the curve beträgt 0,967 (95% CI 0,940-0,984; p=0,0001). Rechts: Sensitivität und Spezifität für die Diagnose Pneumonie durch PCT bei verschiedenen Schwellenwert-Konzentrationen (cut-off [ng/mL]). Größte diagnostische Genauigkeit bei hervorgehobenem Schwellenwert.

## Patentansprüche

1. Verfahren zur *in-vitro* Diagnose von bakterieller Pneumonie mit assoziierter Herzinsuffizienz, **dadurch gekennzeichnet, dass** eine Bestimmung des Markers Procalcitonin oder eine Teilsequenz davon an Proben von einem zu untersuchenden Patienten durchgeführt wird und die Bestimmung des Procalcitonin in einem Bereich von 0,01 ng/ml bis 1 ng/ml mit einem Schwellenwert zwischen 0,03 ng/ml und 0,25 ng/ml durchgeführt wird.

2. Verfahren zur *in-vitro* Diagnose von bakterieller Pneumonie mit assoziierter Herzinsuffizienz gemäß Anspruch 1, **dadurch gekennzeichnet, dass** eine Bestimmung des Markers Procalcitonin oder eine Teilsequenz davon an Proben von einem zu untersuchenden Patienten durchgeführt wird und die Bestimmung des Procalcitonin in einem Bereich von 0,01 ng/ml bis 1 ng/ml mit einem Schwellenwert zwischen 0,03 ng/ml und 0,1 ng/ml durchgeführt wird.

3. Verfahren nach einem der vorherigen Ansprüche, **dadurch gekennzeichnet, dass** die Bestimmung des Procalcitonin in einem Bereich von 0,01 ng/mL bis 1 ng/mL mit einem Schwellenwert von 0,03 ng/mL bis 0,06 ng/mL durchgeführt wird.

4. Verfahren nach einem der vorherigen Ansprüche, wobei der Marker Procalcitonin aus den AS 1-116 und/oder AS 3-116 besteht.

5. Verfahren nach einem der vorherigen Ansprüche, **dadurch gekennzeichnet, dass** zusätzlich eine Bestimmung mindestens eines weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

6. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, und Adhäsionsmolekülen, wie VCAM oder ICAM, GDF-15 oder ST2 ausgewählt ist.

7. Verfahren nach einem der vorherigen Ansprüche, **dadurch gekennzeichnet, dass** der cardiovaskuläre Marker aus mindestens einem Marker aus der Gruppe Kreatinkinase, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin oder pro-Adrenomedullin (proADM) oder jeweils eine Teilsequenz davon ausgewählt ist.

8. Verfahren nach einem der vorherigen Ansprüche, **dadurch gekennzeichnet, dass** der ischämischer Marker aus mindestens einem Marker aus der Gruppe Troponin I und T, CK-MB ausgewählt ist.

9. Verfahren nach einem der vorherigen Ansprüche, **dadurch gekennzeichnet, dass** der neurohormonaler Marker mindestens ein natriuretisches Protein ist, insbesondere ANP (bzw. ANF), proANP, NT-proANP, BNP, proBNP, NT-proBNP oder eine Teilsequenz davon ist.

10. Verfahren nach einem der vorherigen Ansprüche, **dadurch gekennzeichnet, dass** parallele oder simultane Bestimmungen der Marker durchgeführt werden.

11. Verfahren nach einem der vorherigen Ansprüche, **dadurch gekennzeichnet, dass** die Bestimmungen an mindestens einer Patientenprobe durchgeführt werden.

12. Verfahren nach einem der vorherigen Ansprüche, **dadurch gekennzeichnet, dass** die Bestimmungen an einem Analyseautomaten, insbesondere mittels einem Kryptor, durchgeführt werden.

13. Verfahren nach einem der vorherigen Ansprüche, **dadurch gekennzeichnet, dass** die Bestimmungen mittels einem Schnelltest, insbesondere in Einzel- oder Multiparameterbestimmungen durchgeführt werden.

14. Verfahren nach einem der vorherigen Ansprüche, **dadurch gekennzeichnet, dass** die Bestimmungen zur Risikostratifizierung von Patienten erfolgt.

15. Verfahren nach einem der vorherigen Ansprüche, **dadurch gekennzeichnet, dass** die Diagnose zur Stratifizierung von Patienten für klinische Entscheidungen, insbesondere weiterführende Behandlung mittels Arzneimitteln zur Behandlung oder Therapie von Herzinsuffizienz und zur Behandlung oder Therapie von Infektionen bakterieller Pneumonie sowie zur Therapiesteuerung einer Antibiotikabehandlung bakterieller Pneumonie, und/oder insbesondere in der Intensivmedizin oder Notfallmedizin bei bakterieller Pneumonie erfolgt.

16. Verfahren nach einem der vorherigen Ansprüche, **dadurch gekennzeichnet, dass** die Diagnose zur Prophylaxe, zur differentialdiagnostischen Früherkennung und Erkennung, zur Beurteilung des Schweregrades und zur therapiebegleitenden Verlaufsbeurteilung einer bakteriellen Pneumonie mit assoziierter Herzinsuffizienz erfolgt.

## Claims

1. Method for *in-vitro* diagnosis of bacterial pneumonia with associated heart failure, **characterized in that** the marker procalcitonin or a partial sequence thereof is determined on specimens of a patient to be examined, and the procalcitonin is determined in a range from 0.01 ng/ml to 1 ng/ml with a threshold value between 0.03 ng/ml and 0.25 ng/ml.

2. Method for *in-vitro* diagnosis of bacterial pneumonia with associated heart failure according to claim 1, **characterized in that** the marker procalcitonin or a partial sequence thereof is determined on specimens of a patient to be examined, and the procalcitonin is determined in a range from 0.01 ng/ml to 1 ng/ml with a threshold value between 0.03 ng/ml and 0.1 ng/ml.

3. Method according to any of the preceding claims, **characterized in that** the procalcitonin is determined in a range from 0.01 ng/ml to 1 ng/ml with a threshold value between 0.03 ng/ml and 0.06 ng/ml.

4. Method according to any of the preceding claims, wherein the marker procalcitonin consists of AS 1-116 and/or AS 3-116.

5. Method according to any of the preceding claims, **characterized in that** in addition at least one further marker, which is selected from the group of inflammatory markers, cardiovascular markers, neurohormonal markers or ischaemic markers, is determined on a patient to be examined.

6. Method according to any of the preceding claims, **characterized in that** the inflammatory marker consists of at least one marker from the group of C-reactive protein (CRP), cytokines such as TNF-alpha, interleukins such as IL-6, and adhesion molecules such as VCAM or ICAM, GDF-15 or ST2.

7. Method according to any of the preceding claims, **characterized in that** the cardiovascular marker consists of at least one marker from the group creatinkinase, myoglobin, natriuretic protein, in particular ANP (or ANF), proANP, NT-proANP, BNP, proBNP, NT-proBNP or a partial sequence thereof, cardiac troponin, CRP and circulation-regulating (pro)hormones such as pro-gastrin-releasing peptide (proGRP), pro-endothelin-1, pro-leptin, pro-neuropeptid-Y, pro-somatostatin, pro-neuropeptid-YY, pro-opiomelanocortin or pro-adrenomedullin (proADM) or a partial sequence thereof.

8. Method according to any of the preceding claims, **characterized in that** the ischaemic marker consists of at least one marker from the group troponin I and T, CK-MB.

9. Method according to any of the preceding claims, **characterized in that** the neurohormonal marker is at least one natriuretic protein, in particular ANP (or ANF), proANP, NT-proANP, BNP, proBNP, NT-proBNP or a partial sequence thereof.

10. Method according to any of the preceding claims, **characterized in that** the markers are determined in parallel or simultaneously.

11. Method according to any of the preceding claims, **characterized in that** the markers are determined on at least one patient specimen.

12. Method according to any of the preceding claims, **characterized in that** the markers are determined on an analysis machine, in particular by means of a cryptor.

13. Method according to any of the preceding claims, **characterized in that** the markers are determined in a rapid test, in particular in individual or multiparameter determinations.

14. Method according to any of the preceding claims, **characterized in that** the markers are determined to classify the risk to patients.

15. Method according to any of the preceding claims, **characterized in that** the diagnosis is made for classifying patients for clinical decisions, in particular for further treatment by means of drugs for treatment or therapy of heart failure and for treatment or therapy of infections of bacterial pneumonia and for therapy control of an antibiotic treatment of bacterial pneumonia, and/or in particular in the intensive care or emergency treatment of bacterial pneumonia.

16. Method according to any of the preceding claims, **characterized in that** the diagnosis is made for prophylaxis, for differential diagnostic early detection and detection, for assessment of severity and for therapy-accompanying development assessment of a bacterial pneumonia with associated heart failure.

## Revendications

1. Procédé de diagnostic *in vitro* de la pneumonie bactérienne associée à une insuffisance cardiaque, **caractérisé en ce qu'**une détermination du marqueur procalcitonine ou d'une séquence partielle de celui-ci est réalisée sur des échantillons d'un patient à étudier et la détermination de la procalcitonine s'effectue dans une plage allant de 0,01 ng/ml à 1 ng/ml avec un seuil situé entre 0,03 ng/ml et 0,25 ng/ml.

2. Procédé de diagnostic *in vitro* de la pneumonie bactérienne associée à une insuffisance cardiaque selon la revendication 1, **caractérisé en ce qu'**une détermination du marqueur procalcitonine ou d'une séquence partielle de celui-ci est réalisée sur des échantillons d'un patient à étudier et la détermination de la procalcitonine s'effectue dans une plage allant de 0,01 ng/ml à 1 ng/ml avec un seuil situé entre 0,03 ng/ml et 0,1 ng/ml.

3. Procédé selon l'une des revendications précédentes, **caractérisé en ce que** la détermination de la procalcitonine s'effectue dans une plage allant de 0,01 ng/ml à 1 ng/ml avec un seuil situé entre 0,03 ng/ml jusqu'à 0,06 ng/ml.

4. Procédé selon l'une des revendications précédentes, dans lequel le marqueur procalcitonine se compose de AA 1-116 et/ou AA 3-116.

5. Procédé selon l'une des revendications précédentes, **caractérisé en ce que**, en plus, une détermination d'au moins un autre marqueur choisi dans le groupe des marqueurs inflammatoires, des marqueurs cardiovasculaires, des marqueurs neurohormonaux ou des marqueurs ischémiques est réalisée sur un patient à étudier.

6. Procédé selon l'une des revendications précédentes, **caractérisé en ce que** le marqueur inflammatoire est choisi parmi au moins un marqueur du groupe protéine C-réactive (CRP), cytokines, comme par exemple le TNF-alpha, interleukines, comme par exemple l'IL-6, et molécules d'adhésion, comme VCAM ou ICAM, GDF-15 ou ST2.

7. Procédé selon l'une des revendications précédentes, **caractérisé en ce que** le marqueur cardiovasculaire est choisi parmi au moins un marqueur du groupe créatine kinase, myoglobine, protéine natriurétique, en particulier l'ANP (ou ANF), le pro-ANP, le NT-proANP, le BNP, le proBNP, le NT-proBNP ou respectivement une séquence partielle de ceux-ci, la troponine cardiaque, CRP et les (pro)hormones régulant la circulation, comme le peptide libérant pro-gastrine (proGRP), pro-endothéline-1, pro-leptine, pro-neuro-peptide-Y, pro-somatostatine, pro-neuro-peptide-YY, pro-opiomélanocortine ou pro-adrénomédulline (proADM) ou respectivement une séquence partielle de ceux-ci.

8. Procédé selon l'une des revendications précédentes, **caractérisé en ce que** le marqueur ischémique est choisi parmi au moins un marqueur provenant du groupe troponine I et T, CK-MB.

9. Procédé selon l'une des revendications précédentes, **caractérisé en ce que** le marqueur neurohormonal est au moins une protéine natriurétique, en particulier l'ANP (ou ANF), le pro-ANP, le NT-proANP, le BNP, le proBNP, le NT-proBNP ou une séquence partielle de ceux-ci.

10. Procédé selon l'une des revendications précédentes, **caractérisé en ce que** des déterminations parallèles ou simultanées des marqueurs sont réalisées.

11. Procédé selon l'une des revendications précédentes, **caractérisé en ce que** les déterminations sont réalisées sur au moins un échantillon de patient.

12. Procédé selon l'une des revendications précédentes, **caractérisé en ce que** les déterminations sont réalisées sur un automate d'analyse, en particulier au moyen d'un Kryptor.

13. Procédé selon l'une des revendications précédentes, **caractérisé en ce que** les déterminations sont réalisées au moyen d'un test rapide, en particulier en déterminations mono- ou multiparamètres.

14. Procédé selon l'une des revendications précédentes, **caractérisé en ce que** les déterminations se font pour la stratification des risques des patients.

15. Procédé selon l'une des revendications précédentes, **caractérisé en ce que** le diagnostic pour la stratification des patients s'effectue pour des décisions cliniques, en particulier la poursuite du traitement au moyen de médicaments pour le traitement ou la thérapie de l'insuffisance cardiaque et pour le traitement ou la thérapie des infections de pneumonie bactérienne et aussi le contrôle thérapeutique d'un traitement antibiotique de la pneumonie bactérienne et/ou en particulier en médecine de soins intensifs ou en médecine d'urgence en cas de pneumonie bactérienne.

16. Procédé selon l'une des revendications précédentes, **caractérisé en ce que** le diagnostic est effectué pour la prophylaxie, pour l'identification précoce et l'identification par diagnostic différentiel, pour l' évaluation du degré de gravité et pour l'évaluation de la progression accompagnant la thérapie d'une pneumonie bactérienne associée à insuffisance cardiaque.
